# EUROPÄISCHE PATENTSCHRIFT

(11) **EP 2 143 373 B1**
(45) Veröffentlichungstag und Bekanntmachung des Hinweises auf die Patenterteilung: **28.09.2011**
(21) Anmeldenummer: 09164614.1
(22) Anmeldetag: 06.07.2009
(51) Int. Cl.: A61B 1/07, G02B 6/38, G02B 6/40

(54) **Videoendoskop mit schaltbaren Halbleiterlichtquellen**
Video endoscope with switchable semi-conductor light sources
Vidéo-endoscope doté de sources lumineuses semi-conductrices commutables

(30) Priorität: 07.07.2008 DE 102008033506
(43) Veröffentlichungstag der Anmeldung: 13.01.2010
(73) Patentinhaber: Karl Storz GmbH & Co. KG, 78532 Tuttlingen (DE)
(72) Erfinder: Irion, Dr. Klaus-Martin, 78576, Emmingen-Liptingen (DE); Schwarz, Peter, 78532, Tuttlingen (DE)
(74) Vertreter: Witte, Weller & Partner

(56) Entgegenhaltungen:
- WO-A-2006/108143
- CH-A5- 604 193
- DE-A1- 19 621 370
- JP-A- 62 258 408
- US-A- 4 753 501
- US-A- 6 128 426
- US-A1- 2003 185 487

## Beschreibung

Die Erfindung betrifft ein Videoendoskop, mit einem Endoskopteil, das proximalseitig einen mittigen Bildsignalleiter und zumindest einen Lichtleiter aufweist, und mit einem an das Endoskopteil koppelbaren Lichtzuführteil, das distalseitig einen mittigen Bildsignalleiteranschluss sowie eine Lichtzuführung aufweist, die im Koppelbereich koaxial um den Bildsignalleiteranschluss angeordnet ist, wobei Endoskopteil und Lichtzuführteil drehbar zueinander sind.

Ein derartiges Videoendoskop ist aus der DE 39 14 825 C1 bekannt.

Bei derartigen Videoendoskopen, die zwei miteinander koppelbare Bauteile aufweisen, besteht aus ergonomischen Gründen eine Forderung darin, dass beim Koppeln das Bildleit- bzw. das Bildübertragungssystem und das Lichtleit- bzw. Lichtübertragungssystem in einem Vorgang gekoppelt werden. Eine getrennte Ankopplung von Lichtleiterkabel und Kamera an verschiedenen Schnittstellen ist zeitaufwändig und umständlich. Außerdem werden zwei getrennte Kabel notwendig, die meist in unterschiedlichen Richtungen abgehen, was mehr Platz benötigt und mehr Sicht im Operationsfeld verdeckt.

Daher wurden Systeme einer Universalkopplung entwickelt, bei der Licht und Bild in einem gekoppelt werden.

Ein solches Kopplungssystem, wie es von der Anmelderin geschaffen wurde, ist in der DE 197 15 510 A1 beschrieben. Dort steht vom Endoskopteil axial ein erster Zapfen vor, der das bildleitende System enthält, parallel dazu erstreckt sich ein weiterer Zapfen, der das Lichtleitsystem enthält.

Diese beiden Zapfen werden in entsprechende Vertiefungen im Kamerateil eingesteckt, so dass eine exakt ausgerichtete Orientierung von Bild- und Lichtleitsystem bei einem einfachen Kopplungsvorgang sichergestellt wird.

Nachteilig bei diesem System ist, dass Licht und Bild starr, d.h. nicht rotierbar, gekoppelt sind.

Des Weiteren beschreibt die CH604193 eine Vorrichtung zur Beleuchtung und Inspektion von Hohlräumen mit einem bezüglich eines festen Sehrohrs um seine Längsachse drehbaren endoskopischen Sehrohr.

Bei zahlreichen Endoskopen, insbesondere bei starren Endoskopen, erfolgt die Blickrichtung meist nicht exakt in der Längsachse des Schaftes (0°-Blickrichtung), sondern von dieser abweichend, beispielsweise in einer 30°-Blickrichtung. Wird nun bei einem operativen Vorgang dieses Endoskop gedreht, dreht sich auch zwangsläufig das Bild, beispielsweise ein von der Kamera auf einem Monitor visualisiertes Bild, das der Operateur beobachtet. Hat er mit dem Endoskop beispielsweise eine 180°-Drehung im Körper durchgeführt, würde das Bild, das er betrachtet, auf dem Kopf stehen. Da dies aber nicht gewünscht ist, sondern die Operateure das Bild in der aufrecht stehenden Stellung sehen wollen, haben sich sogenannte Bildaufrichtungssysteme entwickelt und durchgesetzt.

Bei der starren Kopplung, wie sie in der DE 197 15 510 A1 beschrieben ist, erfordert dies aufwändige Bildaufrichtungssysteme, wie das beispielsweise in der US 6,097,423 beschrieben ist.

Bei dem eingangs beschriebenen Videoendoskop der DE 39 14 825 C1 sind die beiden gekoppelten Teile, nämlich das Endoskopteil und das Kamerateil, das das Beleuchtungslicht zuführt, drehbar. Das Bildleit- bzw. Bildübertragungssystem ist dabei sowohl im Endoskopteil als auch im Kamerateil mittig zentral angeordnet und an einer Schnittstelle gekoppelt.

Das lichtleitende System des Endoskopteils ist an dieser Schnittstelle so ausgebildet, dass um das mittige Bildleit-/Bildübertragungssystem umfänglich herum ein Ring an Lichtleitern angeordnet ist. Im Beleuchtungslicht-/Kamerateil ist ein entsprechender Ring gleichen Durchmessers vorgesehen, der an der Schnittstelle dem Ring an Lichtleitern des Endoskopteiles gegenüberliegt.

Die Lichtleiter bestehen üblicherweise aus zahlreichen dünnen Lichtfasern, die möglichst gebündelt im Endoskop geführt werden. Diese Lichtleiterfaser müssen zu dem Ring an der Koppelteile aufgespleißt werden. Entsprechendes gilt für das Kamerateil.

Demzufolge können nur solche Endoskopteile an das Kamerateil angekoppelt haben, bei denen der aufgespleißte Ring an Lichtleitern exakt dem Durchmesser des Ringes am Kamerateil entspricht, denn sonst kann kein Beleuchtungslicht übertragen werden.

Bei kleinen Endoskopen mit relativ dünnen Schäften müssten dann die Lichtleiter in einen relativ großen Ring an Lichtleitern an der Schnittstelle aufgespleißt werden, wodurch insbesondere bei kleinkalibrigen Endoskopen keine optimale Adaptionsmöglichkeit besteht, da der erforderliche koaxiale Lichtring bei dünnkalibrigen Endoskopen relativ dünn wird und es zu hohen Koppelverlusten kommt.

Es besteht aber ein Bedarf an Standardkupplungen sowohl für großkalibrige als auch für kleinkalibrige Endoskope, die eine drehbare Kupplung für eine Bildaufrichtung enthalten, die einfach und sicher den Kupplungsvorgang in einem ermöglichen und die außerdem eine Lichtleiterkopplung mit möglichst geringen Koppelverlusten zeigen.

Es ist daher Aufgabe der vorliegenden Erfindung, ein Videoendoskop der eingangs genannten Art dahingehend weiterzuentwickeln, dass eine koaxiale Kopplung von Licht und Bild möglich ist, wobei Kopplungsverluste möglichst gering gehalten werden sollen, und eine mechanisch einfache Drehbarkeit möglich ist.

Erfindungsgemäß wird die Aufgabe dadurch gelöst, dass das Lichtzuführteil mehrere, um den Bildsignalleiteranschluss herum angeordnete lichtabstrahlende Elemente aufweist, die jeweils über schaltbare Halbleiterlichtquellen versorgbar sind, wobei im Bereich der Elemente Positionssensoren angeordnet sind, und dass am Endoskopteil im Bereich des Lichtleiters ein Positionsgeber angeordnet ist, dessen Position in gekoppeltem Zustand durch Positionssensoren erfassbar ist, und dadurch zumindest dem Lichtleiter gegenüberliegende lichtabstrahlende Elemente aktiviert werden.

Der grundlegende Gedanke besteht nun darin, die Kopplung koaxial so auszugestalten, dass die Bildinformationen zentral gekoppelt wird. Die mehreren, um den Bildsignalleiteranschluss herum angeordneten lichtabstrahlenden Elemente sind jeweils individuell ansteuerbar, und zwar in Abhängigkeit von der Position, an der sich beim angekoppelten Endoskopteil Lichtleiter bzw. ein Lichtleiteranschluss befindet. Dazu sind im Bereich der Elemente jeweils Positionssensoren angeordnet, die die Position des Lichtleiters am Endoskopteil über dessen Positionsgeber erfassen. Dadurch ist es möglich, beim Endoskopteil den Lichtleiteranschluss oder auch ggf. mehrere Lichtleiteranschlüsse, an Stellen im Kopplungsbereich zu positionieren, die für die Konstruktion des Endoskopteiles am günstigsten sind.

Im Bereich der Stelle, an der am Endoskopteil im Kopplungsbereich ein Lichtleiter, meist ein Lichtleiterbündel, in einen Lichtleiteranschluss mündet, ist ein entsprechender Positionsgeber vorhanden. Diese Position wird beim Koppeln von dem Lichtzuführteil erfasst und es werden zumindest diejenigen lichtabstrahlenden Elemente des Lichtzuführteils aktiviert, die dem oder den Lichtleitern an der Kopplungsstelle gegenüberliegen.

Halbleiterlichtquellen, vor allem LEDs und OLEDs, sind preiswert sowie in gewünschten geometrischen Formen herstellbar und können schnell geschaltet werden. Daher können flächige lichtabstrahlende Elemente bereitgestellt werden, die um den zentralen Bildsignalleiteranschluss, auf der gesamten Kopplungsfläche verteilt, herum angeordnet werden.

Wird das Endoskopteil gedreht, also seine Position relativ zum Lichtzuführteil verändert, wird dies durch das Lichtzuführteil detektiert und es werden die lichtabstrahlenden Elemente aktiviert, die nunmehr dem Lichtleiter gegenüberliegen. So ist sichergestellt, dass in jedem beliebigen Drehzustand zumindest diejenigen lichtabstrahlenden Elemente aktiviert sind, die dem Querschnitt des Lichtleiteranschlusses des Endoskopes gegenüberliegen. Darüber hinaus können auch die Übergangsbrücken noch ausgeleuchtet werden.

Dies hat nicht nur den Vorteil, dass in jeder Drehposition eine optimale Lichtquelle mit geringen Koppelverlusten zur Verfügung steht, sondern dass beispielsweise auch erkannt werden kann, in welchem radialen Abstand der Lichtleiter zum mittigen Bildsignalleiteranschluss liegt, also ob Endoskope unterschiedlichen Kalibers oder mit unterschiedlichen Anordnungen des Lichtleiteranschlusses an der Koppelstelle vorliegen.

Neben der Lichtkopplung erfolgt mittig die Bildkopplung. Der Bildsignalleiter des Endoskopteiles ist bei optischer Bildführung ein starres Stablinsensystem oder ein flexibles Faserbildleitsystems.

Bei Videoendoskopen kann das Bild über einen elektrischen Bildwandler in ein elektrisches Signal umgewandelt werden. Als Bildwandler eignen sich insbesondere Festkörpersensoren wie CCD-Sensoren oder CMOS-Sensoren.

Der Bildsignalleiteranschluss des Lichtzuführteils ist so ausgebildet, dass, unabhängig von der Ausgestaltung der Lichtkopplung des Endoskopteiles, dessen Bildsignal weitergeleitet und/oder umgewandelt werden kann. Im Lichtzuführteil ist daher ein elektrischer Bildwandler vorhanden, der ein vom Endoskopteil geliefertes optisches Bildsignal in ein elektrisches Bildsignal umwandelt. Es ist somit ein Kamerateil.

Damit auch Videoendoskope angekoppelt werden können, kann zusätzlich oder alternativ ein elektrischer Signalkanal vorhanden sein, der elektrische Bildsignale des Endoskopteiles weiterleitet.

Der Bildsignalleiteranschluss des Lichtzuführteiles umfasst daher einen optischen Bildsignalleiter mit elektrischem Bildwandler und einen elektrischen Bildsignalleiter.

Dies eröffnet eine hohe Variabilität und Flexibilität bezüglich des Einsatzbereiches von Endoskopteil einerseits und Lichtzuführteil andererseits, die aber dennoch miteinander standardisiert gekoppelt werden können, wobei sichergestellt ist, dass eine streuverlustarme Lichtkopplung möglich ist.

Ist beispielsweise ein Lichtleiteranschluss am Endoskopteil mit einem relativ großen Querschnitt vorhanden, so dass mehrere lichtabstrahlenden Elemente überdeckt werden, können alle diese Elemente zur Lichtabgabe aktiviert werden, die in dem Querschnitt des Lichtleiters liegen. Beim Drehen der Kupplung ist dann auch immer sichergestellt, dass in jeder beliebigen Drehposition zumindest all diejenigen lichtabstrahlenden Elemente aktiviert sind, die im Bereich des Querschnittes des oder der Lichtleiteranschlüsse des Endoskopteiles liegen. Damit ist immer eine optimale Beleuchtungsstärke vorhanden, die in den Lichtleiter eingekoppelt wird. Dies ist durch das Zusammenspiel der Positionssensoren am Lichtzuführteil und dem Positionsgeber im Bereich des Lichtleiters am Endoskopteil zu realisieren.

In einer weiteren Ausgestaltung werden nur diejenigen lichtabstrahlenden Elemente aktiviert, die im Querschnittsbereich eines Lichtleiters des Endoskopteiles liegen.

Jedes lichtabstrahlende Element strahlt auch zugleich Wärme ab. Werden nur diejenigen lichtabstrahlenden Elemente aktiviert, die im Querschnittsbereich des Lichtleiters im Endoskopteil liegen, wird also nur in dem Bereich am Lichtzuführteil Licht abgestrahlt. Dadurch können nicht nur unnötige Lichtverluste vermieden werden, sondern auch eine unnötige Wärmestrahlung, die zu einer Erwärmung im Kopplungsbereich führen könnte, die andere Bauelemente negativ beeinflusst, oder die Handhabung durch den Operateur stört.

Außerdem ist dies energiebilanzmäßig günstig. Dies eröffnet auch weitere Möglichkeiten im Zusammenhang mit geordneten Lichtleiteranordnungen im Endoskopteil, so dass es prinzipiell möglich ist, eine strukturierte Beleuchtung bzw. Ausleuchtung des Bildfeldes zu schaffen.

In einer weiteren Ausgestaltung der Erfindung sind die einzelnen lichtabstrahlenden Elemente segmentartig ausgebildet.

Diese Maßnahme hat den Vorteil, dass die mehreren Elemente zu Gruppen oder Mustern zusammengesetzt werden können, um Bereiche abzudecken, in denen die Lichtleiter des Endoskopteiles zum Liegen kommen können.

In einer weiteren Ausgestaltung der Erfindung sind die lichtabstrahlenden Elemente zu zumindest einem Ring zusammengesetzt.

Diese Maßnahme hat den Vorteil, dass bei einer bestimmten radialen Position eines Lichtleiters an der Kupplungsfläche des Endoskops, in dessen gesamten 360°-Drehbereich, eine lückenlose Lichtübertragung möglich ist. Durch entsprechende Ausformung der segmentartigen lichtabstrahlenden Elemente können diese zu durchgehenden Beleuchtungsringen zusammengesetzt werden. Dabei können auch mehrere konzentrische Beleuchtungsringe realisiert werden, so dass beispielsweise bei einem Endoskopteil, bei dem der Lichtleiteranschluss in einem relativ großen radialen Abstand zur mittigen Bildleiteranschluss angeordnet ist, nur ein entsprechend radial äußerer Beleuchtungsring des Lichtzuführteiles aktiviert werden muss.

In einer weiteren Ausgestaltung der Erfindung sind die schaltbaren Halbleiterlichtquellen ausgewählt aus der Gruppe bestehend aus LEDs, OLEDs, Diodenlaser oder Kombinationen davon.

Wie bereits erwähnt, sind diese Halbleiterlichtquellen sehr kostengünstig und können sehr schnell geschaltet werden, ohne dass dadurch die Lebensdauer wesentlich beeinträchtigt wird. Diese sind in beliebigen geometrischen Formen herstellbar, somit beispielsweise in Kreisringabschnitten, so dass die einzelnen Elemente zu Beleuchtungsringen zusammengesetzt werden können, die um den mittigen Bildsignalleiteranschluss herum verlaufen.

In einer weiteren Ausgestaltung der Erfindung stellt die schaltbare Halbleiterlichtquelle unmittelbar das lichtabstrahlende Element dar.

Diese Maßnahme hat den Vorteil, dass konstruktiv sehr einfach diese lichtabstrahlenden Elemente aufgebaut sind, indem diese nämlich direkt an der Stirnfläche im Bereich der Kupplungsfläche am Lichtzuführteil montiert werden.

In einer weiteren Ausgestaltung der Erfindung sind die schaltbaren Halbleiterquellen im Abstand zu dem endseitigen lichtabstrahlenden Element angeordnet und sind mit diesem über Lichtleiter verbunden.

Diese Maßnahme hat den Vorteil, dass die die wärmeabstrahlende Halbleiterlichtquelle etwas abseits der eigentlichen Kupplungsstelle liegt, so dass sie nicht nur vor mechanischen Einflüssen im Bereich der Kupplungsstelle geschützt ist, sondern auch die Möglichkeit eröffnet ist, an dieser abseitigen Stelle die Lichtquelle zu kühlen. Man muss allerdings dafür sorgen, dass möglichst geringe Kopplungsverluste bei der Weiterleitung des Lichtes an die an der endseitigen Kupplung angeordneten lichtabstrahlenden Elemente auftreten.

Außerdem eröffnet diese Möglichkeit durch entsprechende Ausgestaltung der Lichtleiter und der lichtabstrahlenden Elemente Einfluss auf das von der Lichtquelle erzeugte Licht zu nehmen, beispielsweise im Sinne einer Filterung, einer Polarisierung oder dergleichen. So können die lichtaufnehmenden und weiterleitenden Elemente aus Lichtfasern unterschiedlicher numerischer Apertur bestehen, wodurch das Bildfeld unterschiedlich ausgeleuchtet werden kann, so dass z.B. eine 3D-Tiefenempfindung durch Schattenbildung erzeugt werden kann.

In einer weiteren Ausgestaltung der Erfindung strahlen die lichtabstrahlenden Elemente Licht unterschiedlicher Farbe ab.

Diese Möglichkeit eröffnet farblich unterschiedliche Illuminationen und Beleuchtungen bei der photodynamischen Diagnose. Dabei wird dem zu untersuchenden Probanden vorher ein Mittel verabreicht, das beispielsweise von einem Tumorgewebe stärker absorbiert wird, als von nicht vom Tumor befallenen Gewebe. Durch Bestrahlen mit einem bestimmten Wellenlängenbereich einer bestimmten Farbe kann dieser Tumor zu einer besonders intensiven Fluoreszenz angeregt werden.

Somit eröffnet die Konstruktion nicht nur standardisierte und sichere Kupplungen, sondern eröffnet zugleich Untersuchungsmaßnahmen, wie die photodynamische Diagnose.

In einer weiteren Ausgestaltung der Erfindung sind die Halbleiterlichtquellen in einer separaten Aperatur aufgenommen.

Diese Maßnahme hat den Vorteil, dass im Falle von hochenergetischem Licht die Halbleiterlichtquellen soweit abseits von der Koppelstelle sind, dass die damit verbundene Wärmeabstrahlung stark herabgesetzt wird. Außerdem ist es möglich, die Halbleiterlichtquellen entsprechend zu kühlen, auch um beispielsweise deren Lebensdauer zu erhöhen.

Entsprechend ist in einer weiteren Ausgestaltung vorgesehen, eine Kühlung zum Kühlen der Halbleiterlichtquellen vorzusehen.

In einer weiteren Ausgestaltung der Erfindung sind die Positionssensoren und Positionsgeber als Hall-Sensoren ausgebildet.

Die Erfassung und Detektion einer Position basierend auf dem Prinzip der Hall-Sensoren haben einen weiten Einsatz in der Technik gefunden und es stehen somit kostengünstig solche Sensoren zur Verfügung. Damit kann eine sichere Positionserfassung durchgeführt werden, so dass die jeweils optimale Anstrahlung der Lichtleiter im Endoskopteil sichergestellt ist.

Es können selbstverständlich auch andere Lagesensoren auf Basis von optischen Effekten oder sonstigen Effekten ausgewählt werden. Wie bereits erwähnt, sind die Hall-Sensoren aber technisch sehr ausgereift und günstig zur Verfügung stehend.

In einer weiteren Ausgestaltung der Erfindung weist das Endoskopteil ein Identifikationselement auf, welches über ein im Lichtzuführteil angeordnetes Leseelement auslesbar ist.

Diese Maßnahme hat den Vorteil, dass über die Identifikation der Typ des Endoskopteiles bestimmt werden kann und somit z.B. die Größe, die Lage und die Anzahl der lichtaufnehmenden Bündel kodiert werden kann. Dadurch ist eine optimale Ansteuerung der zu schaltenden Lichtelemente gegeben.

In einer weiteren Ausgestaltung der Erfindung ist ein Rastmechanismus vorgesehen, der ein Verrasten von Endoskopteil und Lichtzuführteil in gekoppeltem Zustand bewirkt.

Diese Maßnahme hat den Vorteil, dass durch den Rastmechanismus die einmal aneinandergekoppelten Teile axial lagefixiert, jedoch drehbar miteinander verbunden sind.

In einer weiteren Ausgestaltung der Erfindung ist der Rastmechanismus derart ausgebildet, dass ein Drehen des Endoskopteiles um das Lichtzuführteil in Positionen mit äquidistanten Winkelstellungen möglich ist.

Diese Maßnahme hat den Vorteil, dass über den Rastmechanismus Positionen optimaler Lichtübertragung eingenommen werden können.

In einer weiteren Ausgestaltung der Erfindung weist das Endoskopteil ein Steuerelement auf, über das es bei erfasstem Lichtzuführteil um dieses drehbar ist.

Diese Maßnahme hat den Vorteil, dass das Lichtzuführteil mit einer Hand gehalten werden kann, und mit einem Finger der Hand kann über das Steuerelement ein Drehen des Endoskopteiles durchgeführt werden. Dies erlaubt eine besonders ergonomische Handhabung der gekoppelten Teile, beispielsweise bei einer manuell gewünschten Bildaufrichtung.

In einer weiteren Ausgestaltung der Erfindung ist das Lichtzuführteil als Kamerateil mit einem elektrischen Bildwandler ausgebildet.

Diese Maßnahme hat den Vorteil, dass das Lichtzuführteil auch das Kamerateil darstellt, also das optische Signal in ein elektrisches Signal umwandelt. Diese Maßnahme hat besonders den Vorteil, dass an ein solches Kamerateil standardmäßige Endoskope mit optischer Lichtleitung, insbesondere die standardmäßigen starren Endoskope mit Linsenlichtleitersystemen angekoppelt werden können.

In einer weiteren Ausgestaltung der Erfindung ist im Endoskopteil ein elektrischer Bildwandler integriert, und im Lichtzuführteil ist ein Steckerteil vorgesehen, um das elektrische Bildsignal weiterzuleiten.

Diese Maßnahme hat den Vorteil, dass das Endoskop als Kamerateil, also als Videoendoskopteil mit integrierten Bildsensoren, ausgestaltet ist. Die Bildinformation des Endoskopteils wird bereits als elektrisches Signal dem Lichtzuführteil zugeführt. Es ist dann eine entsprechende Kupplungsstelle vorzusehen. Dies kann standardmäßig vorgesehen sein oder durch ein zusätzliches Steckersystem realisiert werden.

In einer weiteren Ausgestaltung der Erfindung ist das Lichtzuführteil als Standgerät ausgebildet, in das das Endoskopteil einsteckbar ist.

Diese Maßnahme ist vor allem bei semiflexiblen oder flexiblen Endoskopen vorteilhaft. Dies eröffnet auch die Möglichkeit, zusätzlich Maßnahmen vorzusehen, die beispielsweise ein Drehen des eingesteckten Endoskopteiles möglich machen, um ganz gezielt auf eine andere Halbleiterlichtquelle umzuschalten, die beispielsweise eine andere Farbtemperatur aufweist, eine andere numerische Aperatur, oder auch schlicht als Ersatzlichtquelle im Rahmen eines Teildefektes dienen kann.

In einer weiteren Ausgestaltung der Erfindung ist im Lichtzuführteil ein Antrieb vorhanden, durch den ein angekoppeltes Endoskopteil drehbar ist.

Diese Maßnahme hat den Vorteil, dass eine automatisierte Drehung des Endoskopteiles um seine Längsachse möglich ist. Dies eröffnet beispielsweise durch Betätigung des Antriebes einen Rundumblick um 360° bei einer Optik, die eine von 0° verschiedene Blickrichtung aufweist, um eine Orientierung durchzuführen.

In einer weiteren Ausgestaltung weist das Endoskopteil einen mittigen, nach proximal vorstehenden Zapfen auf, in dem der Bildsignalleiter aufgenommen ist.

Diese Maßnahme hat den Vorteil, dass der Kopplungsvorgang mechanisch einfach gezielt und sicher durchführbar ist, indem nämlich der vorstehende Zapfen des Endoskopes gezielt an das Lichtzuführteil angesetzt werden kann.

Dazu ist in einer weiteren Ausgestaltung vorteilhaft, dass der Bildsignalleiteranschluss des Lichtzuführteils eine Aushöhlung aufweist, in die der vorstehende Zapfen einsteckbar ist.

Diese Maßnahme hat den Vorteil, dass dieser Vorgang ohne größere Aufmerksamkeit zielgerecht und sicher durchführbar ist. Außerdem ist dann die Bildübertragungs- bzw. Bildkoppelstelle abseits von der Lichtkoppelstelle gelegen, so dass eine Beeinflussung des übertragenen Bildes, insbesondere bei optischen Systemen, durch Beleuchtungsstreulicht ausgeschlossen werden kann.

Der Zapfen eröffnet weitere vorteilhafte Möglichkeiten, nämlich beispielsweise an diesem ein Identifikationselement vorzusehen, das beim Einstecken an einem entsprechenden Leseelement in der Aushöhlung vorbeigeführt wird. An diesem Zapfen können auch Klemmelemente oder dergleichen angebracht werden, um eine zusätzliche Abrutschsicherung, wenn beispielsweise der Rastmechanismus noch nicht eingerastet ist, zu bewerkstelligen.

Außerdem kann der Zapfen zugleich als Führung bei der Drehbewegung dienen, so dass diesbezüglich keine weiteren Maßnahmen erforderlich sind.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Die Erfindung wird nachfolgend anhand einiger ausgewählter Ausführungsbeispiele im Zusammenhang mit den beiliegenden Zeichnungen näher erläutert und beschrieben. Es zeigen:
- Figur 1: eine perspektivische Ansicht auf das distale Kopplungsende eines ersten Ausführungsbeispiels eines Lichtzuführteils,
- Figur 2: eine perspektivische Ansicht des proximalen Kopplungsendes eines Endoskopteiles, das mit dem Lichtzuführteil von Figur 1 zu einem Videoendoskop gekoppelt werden kann,
- Figur 3: eine stirnseitige Ansicht des distalen Kopplungsendes eines zweiten Ausführungsbeispieles eines Lichtzuführteiles,
- Figur 4: eine entsprechende proximale endseitige stirnseitige Ansicht des Kopplungsbereichs eines Endoskopteiles, das mit dem Lichtzuführteil von Figur 3 zu einem zweiten Ausführungsbeispiel eines Videoen- doskopes gekoppelt werden kann, und
- Figur 5: stark schematisiert ein drittes Ausführungsbeispiels eines Video- endoskopes, wobei das Lichtzuführteil als Standgerät ausgebildet ist und das Endoskopteil als flexibles Endoskop.

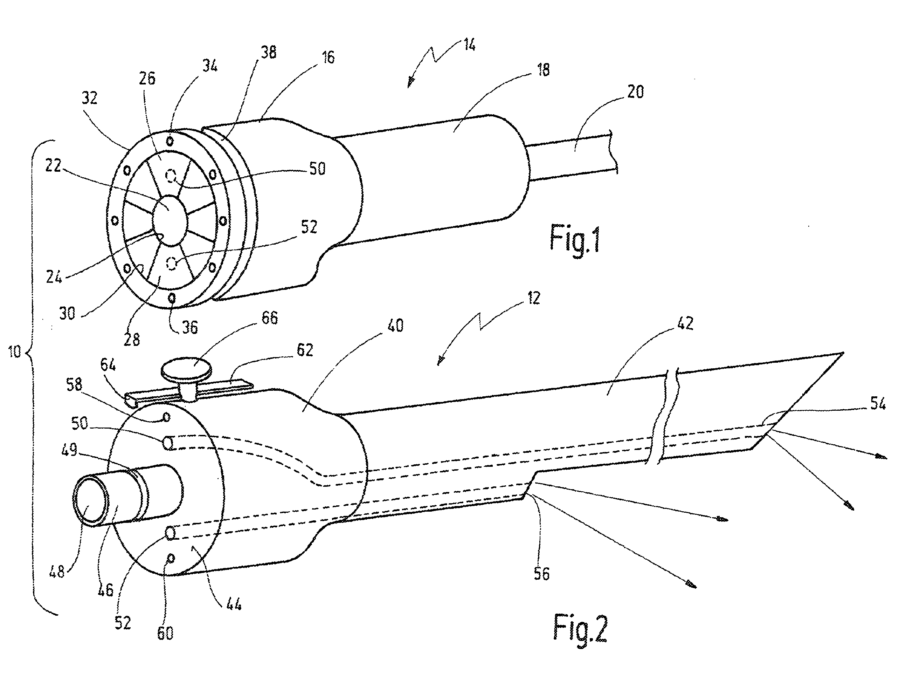

Ein in den Figuren 1 und 2 dargestelltes erstes Ausführungsbeispiel eines Videoendoskopes ist in seiner Gesamtheit mit der Bezugziffer 10 bezeichnet.

Das Videoendoskop 10 besteht aus einem Endoskopteil 12 und einem Lichtzuführteil 14.

Das Lichtzuführteil 14 weist ein Gehäuse 16 auf, das proximalseitig eine etwa stabförmige Handhabe 18 aufweist, über die das Lichtzuführteil 14 bzw. das Videoendoskop 10 von Hand ergriffen werden kann. Proximalseitig werden Kabel 20 abgeführt, die die nachfolgend zu beschreibenden Bauteile elektrisch versorgen oder verbinden.

In Figur 1 ist distalseitig eine ebene, etwa kreisrunde Stirnfläche ersichtlich, die mittig einen Bildsignalleiteranschluss 22 aufweist.

Der Bildsignalleiteranschluss 22 weist eine etwa sacklochförmige Aushöhlung 24 auf, deren Sinn und Zweck später beschrieben wird.

Um die Aushöhlung 24 herum sind insgesamt acht einzelne segmentartige, lichtstrahlende Elemente angeordnet, wobei der Übersicht halber nur zwei diametral gegenüberliegende Elemente 26 und 28 mit Bezugsziffern bezeichnet sind. Die geometrische Ausgestaltung der acht Elemente ist derart, dass sie zusammengesetzt einen Beleuchtungsring 30 ergeben. In dem hier dargestellten Ausführungsbeispiel sind die acht Elemente LEDs, die jeweils einzeln und unabhängig voneinander geschaltet werden können, d.h. zur Lichtabstrahlung aktiviert werden können.

Um den Beleuchtungsring 30 herum ist ein weiterer Ringkörper 32 vorhanden, in dem insgesamt acht Positionssensoren eingesetzt sind, wobei auch hier der Übersicht halber nur die beiden diametral gegenüberliegenden Positionssensoren 34 und 36 mit Bezugsziffern versehen sind. Die Verteilung und Anordnung der acht Positionssensoren ist derart, dass diese jeweils etwa mittig im Bereich der äußeren Umfangskante des jeweiligen lichtabstrahlenden Elements zum Liegen kommen.

Anders ausgedrückt, im Bereich jedes der lichtabstrahlenden acht Elemente ist auch ein Positionssensor angeordnet. Der Sinn und Zweck sowie die schaltungstechnische Funktion wird nachfolgend näher erläutert.

Das Lichtzuführteil 14 ist im dargestellten Ausführungsbeispiel als Kamerateil ausgebildet, d.h. im Bereich des Bodens der sacklochartigen Aushöhlung 24 des Bildsignalleiteranschlusses 22 ist ein elektrischer Bildwandler angeordnet, der ein über den Bildsignalleiteranschluss 22 zugeführtes optisches Bild in ein elektrisches Signal umwandelt. Dieses wird dann über das Kabel 20 weitergeleitet und beispielsweise in einem hier nicht dargestellten Monitor visualisiert.

Im Gehäuse 16 des Lichtzuführteiles 14 ist, vom distalen Ende nach proximal beabstandet, eine umfängliche Nut 38 eingeschnitten, in deren Boden umfänglich gleichmäßig verteilt Vertiefungen vorgesehen sind, in diesem Fall acht umfänglich gleichmäßig verteilte Vertiefungen. Der Sinn und Zweck wird ebenfalls später erläutert.

Das in Figur 2 dargestellte Endoskopteil 12 ist als starres Endoskop ausgebildet.

Das Endoskopteil 12 weist einen Kopf 40 auf, der in einen lang erstreckten starren Schaft 42 übergeht.

Das Endoskopteil 12 weist eine proximalseitige Endfläche 44 auf, von der mittig ein Zapfen 46 nach proximal vorspringt. Die Länge und der Durchmesser des Zapfens 46 ist so gewählt, dass dieser passend in die Aushöhlung 24 des Bildsignalleiteranschlusses 22 des Lichtzuführteils 14 eingeschoben werden kann. Im Inneren des Endoskopteiles 40 setzt sich der Zapfen als Optikkanal fort, der bis zum distalen Ende des Endoskopteiles 12 reicht. Wie bei starren Endoskopen üblich ist darin eine Optik eingesetzt, die aus entsprechend endseitigen Fenstern und Linsen, insbesondere Stablinsen, zusammengesetzt ist.

Diese Optik bildet einen Bildsignalleiter 48 und leitet ein optisches Bildsignal weiter.

Wie ferner aus Figur 2 ist zu erkennen, weist die Endfläche 44 in etwa eine Größe auf, die der gegenüberliegenden Endfläche des Lichtzuführteiles 14 entspricht.

In dem dargestellten Ausführungsbeispiel des Endoskopteiles 12 sind zwei Lichtleiter 50 und 52 von der proximalen Endfläche 44 zu zwei Lichtabstrahlstellen 54 und 56 geführt.

Die Lichtleiter 50 und 52 sind jeweils aus einem Bündel an Glasfasern zusammengesetzt, wie das im Endoskopbau üblich ist.

Die proximalseitigen Enden der Lichtleiter 50 und 52 sind so angeordnet, dass sie diametral gegenüberliegend vom Zapfen 46 beabstandet angeordnet sind.

Radial noch etwas weiter außen liegend sind zwei Positionsgeber 58 und 60 angeordnet, deren radialer Abstand von der Mitte so gewählt ist, dass dieser dem radialen Abstand der Positionssensoren 34 und 36 des Lichtzuführteiles 14 entspricht.

An der Außenseite des Kopfes 40 des Endoskopteiles 12 ist ein Teil eines Rastmechanismus angeordnet, nämlich eine Hebelraste 62, die eine radial nach innen vorstehende Hebelraste 62 vorweist, die so ausgebildet ist, dass diese in die umfängliche Nut 38 am Lichtzuführteil 14 eintreten kann bzw. noch zusätzlich in die darin vorgesehenen zuvor erwähnten Vertiefungen. Mittig an der Hebelraste 62 ist noch ein Steuerelement 66 vorgesehen, dessen Funktion ebenfalls später erläutert wird.

Um die Außenseite des Zapfens 46 ist ein ringförmiges Identifikationselement 49 gelegt, beispielsweise in Form eines Barcodes. Entsprechend ist an der Innenseite der Aushöhlung 24 des Bildsignalleiteranschlusses 22 ein hier nicht ersichtliches Leseelement angeordnet, das den Barcode des Identifikationselementes 49 lesen kann. Zum Kuppeln der Bauteile Endoskopteil 12 und Lichtzuführteil 14 zu dem Videoendoskop 10 wird beispielsweise das Lichtzuführteil 14 über dessen Handhabe 18 mit einer Hand ergriffen. Mit der anderen Hand wird das Endoskopteil 12 ergriffen und dessen Zapfen 49 wird in die Aushöhlung 24 so weit eingeschoben, bis die Rastnase 64 der Hebelraste 62 in die umfängliche Nut 38 einrastet.

Bei diesem Einschieben wird das Identifikationselement 49 an dem Leseelement vorbeigeführt, dort abgelesen, so dass über das Lichtzuführteil 14 die Information erfasst werden kann, um welche Art Endoskopteil 12 und ggf. auch von welchem Hersteller es sich handelt. Beinhaltet diese Information beispielsweise, dass es sich bei dem Endoskopteil 12 um ein solches Endoskopteil handelt, das zwei diametral gegenüberliegende Lichtleiter 50 und 52 aufweist, und ist einer Steuerung des Lichtzuführteils 14 diese Information zugänglich, so können dort schaltungstechnische Maßnahmen ergriffen werden, die das Aktivieren von zwei diametral gegenüberliegenden lichtabstrahlenden Elementen freigibt, vorprogrammiert oder sonstwie verarbeitet.

Werden Endoskopteil 12 und Lichtzuführteil 14 genau in der Ausrichtung von Figuren 1 und 2 miteinander gekoppelt, kommt das proximale Ende des Lichtleiters 50 im Bereich des lichtabstrahlenden Elementes 26 zum Liegen und der Lichtleiter 52 in dem Bereich des lichtabstrahlenden Elementes 28, wie es in Figur 1 durch gestrichelte Kreise angedeutet ist. Der Positionsgeber 58 des Endoskopteiles 12 kommt dabei im Bereich des Positionssensors 34 des Lichtzuführteiles 14 zum Liegen. Entsprechend kommt der Positionsgeber 60 im Bereich des Positionssensors 36 zum Liegen.

Die Positionssensoren und die Positionsgeber sind als sogenannte Hall-Sensoren ausgebildet. Dazu bestehen beispielsweise die beiden Positionsgeber 58 und 60 aus Permanentmagneten, die Positionssensoren 34 und 36 erzeugen ein elektrisches Feld. Beim Koppeln nähern sich die Positionsgeber 58 bzw. 60 dem elektrischen Feld der Positionssensoren, dies kann detektiert werden. Die Schaltung ist nun so gewählt, dass genau die beiden lichtabstrahlenden Elemente 26 und 28 aktiviert werden, in deren Bereich die Lichtleiter 50 und 52 nach dem Koppeln zum Liegen kommen. D.h., es wird effektiv nur dort Licht erzeugt und abgestrahlt, wo sich die Lichteingangsseiten der Lichtleiter 50 und 52 des Endoskopteiles 12 befinden.

Aufgrund der dargestellten Konstruktion ist das Endoskopteil 12 um die Längsachse des Lichtzuführteils 14 drehbar. Diese Drehbarkeit wird zum einen durch den mittigen zentralen Zapfen 46 gut geführt, zur Steuerung kann ein Finger der Hand, die die Handhabe 18 ergriffen hat, auf das Steuerelement 66 gelegt werden und über dieses die Drehbewegung durchgeführt werden. Die Rastnase 64 der Hebelraste 62 läuft dann in der umfänglichen Nut 38.

Dieses Drehen ist beispielsweise dann erwünscht, wenn das Endoskopteil 12 wie im dargestellten Ausführungsbeispiel nicht ein Endoskop mit geradliniger, sprich 0°-Blickrichtung, sondern mit einer abweichenden, beispielsweise einer 30°-Blickrichtung ist.

Diese Drehbarkeit ermöglicht eine gewünschte Bildaufrichtung des von dem Lichtzuführteil 14 erzeugten Videobilds, wenn das Endoskopteil 12 um seine Längsachse gedreht wurde. Dies ist erwünscht, d.h. selbst wenn das Endoskopteil 12 gedreht wurde, wird auf dem Monitor ein aufrecht stehendes Bild dargestellt.

Durch die zuvor beschriebenen Vertiefungen im Boden der umfänglichen Nut 38 können ganz bestimmte äquidistante Winkelstellungen erzielt werden.

Ein besonderer Vorteil der Erfindung drückt sich nun darin aus, dass bei einem solchen Verdrehen dieser Vorgang erfasst werden kann und beleuchtungstechnisch entsprechend darauf reagiert werden kann.

Befinden sich die Enden der Lichtleiter 50 und 52 in der in Figur 1 dargestellten Position und wird nun das angekoppelte Endoskopteil 12 gedreht, in der Ansicht von Figur 1 im Uhrzeigersinn, wird der Lichtleiter 50 den Bereich des lichtabstrahlenden Elementes 26 verlassen und in das im Uhrzeigersinn nächst daneben gelegene Element eintreten.

Dies wird dadurch erfasst, da der Positionsgeber 58 seine vis-à-vis-Position gegenüber dem Positionssensor 34 verlässt und sich dem im Uhrzeigersinn nächstgelegenen Positionssensor nähert. Dies kann wieder durch die Ausgestaltung als Hall-Sensoren erfasst werden, so dass nach einer erfolgten Drehung, beispielsweise um 45°, dann nicht mehr die beiden lichtabstrahlenden Elemente 26 und 28 aktiviert sind, sondern das um 45° in Uhrzeigerrichtung winkelversetzte nächste lichtabstrahlende Elementenpaar.

Dies demonstriert die effektive, ergonomische sowie ökonomische Lichtzuführung des Videoendoskopes 10.

Aus Figur 1 ist ersichtlich, dass bei dieser Drehbewegung ein Zwischenzustand resultieren kann, bei dem der Querschnitt des Lichtleiters 50 sich noch zum Teil im Bereich des lichtabstrahlenden Elementes 26 befindet, zum Teil aber schon das umfänglich nächste Element belegt. Dieser Zwischenzustand kann nun so gehandhabt werden, dass in diesem Übergangsbereich die beiden benachbarten lichtabstrahlenden Elemente aktiviert werden, also sowohl das Element 26 als auch dessen benachbartes Element. Steuerungstechnisch ist das dadurch zu realisieren, dass in diesem Übergangszustand der Positionsgeber 58 gerade den Detektionsbereich des Positionssensors 34 verlassen hat und in den Detektionsbereich des umfänglich nächsten Positionssensors eintritt.

Nach einem Einsatz können die beiden Bauelemente des Videoendoskopes 10 ganz einfach dadurch entkoppelt werden, dass die Hebelraste 62 auf der der Rastnase 64 gegenüberliegenden Seite gedrückt wird, dadurch hebt die Rastnase 64 aus der Nut 38 ab, und Endoskopteil 12 und Lichtzuführteil 14 können voneinander getrennt bzw. voneinander abgezogen werden.

Diese Arretiermöglichkeit ist nur eine Möglichkeit, es kann, falls das gewünscht ist, auch eine Verrastung zwischen der Außenseite des Zapfens 46 und der Innenseite der Aushöhlung 24 bewerkstelligt werden, so dass die Außenseite nicht durch den Rastmechanismus räumlich belegt wird. Diese Rasten können Feder- oder Kugelrasten oder dergleichen sein.

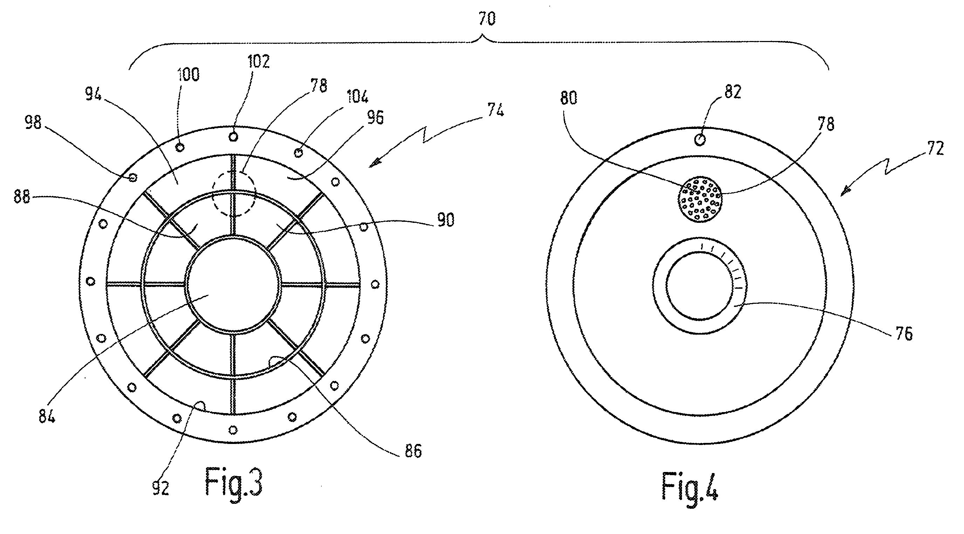

In den Figuren 3 und 4 ist ein weiteres Ausführungsbeispiel eines Videoendoskopes dargestellt, das in seiner Gesamtheit mit der Bezugsziffer 70 versehen ist.

Auch dieses Videoendoskop 70 weist ein Endoskopteil 72 und ein Lichtzuführteil 74 auf. In der Darstellung von Figuren 3 und 4 sind die jeweils diese kuppelseitigen Enden der Bauteile Endoskopteil 72 und Lichtzuführteil 74 ersichtlich.

Vom Endoskopteil 72 steht, wie zuvor beschrieben, der mittige Zapfen 76 vor, in dem die Optik aufgenommen ist.

In diesem Ausführungsbeispiel ist an dem Endoskopteil 72 nur ein einziger Lichtleiter 78 vorhanden, der aus einem Faserbündel 80 an einzelnen Lichtleitfasern zusammengesetzt ist. Auch hier ist am äußeren Rand ein Positionsgeber 82 vorhanden.

Wie aus Figur 3 ersichtlich, weist das Lichtzuführteil 74 wieder eine mittige Aushöhlung 84 auf, in die der Zapfen 76 passend einschiebbar ist.

Um die Aushöhlung 84 ist ein erster Ring 86 vorhanden, der aus acht einzelnen, segmentartigen lichtabstrahlenden Elementen zusammengesetzt ist, wobei hier nur die beiden lichtabstrahlenden Elemente 88 und 90 mit einer Bezugsziffer versehen sind.

Um den ersten Beleuchtungsring ist koaxial ein zweiter Ring 92 gelegt, der aus acht einzelnen, segmentartigen lichtabstrahlenden Elementen zusammengesetzt ist, wobei hier ebenfalls nur die beiden Elemente 94 und 96 mit einem Bezugszeichen versehen sind.

Um den äußeren Beleuchtungsring herum sind insgesamt sechzehn Positionssensoren angeordnet, wobei hier nur die Positionssensoren 98, 100, 102 und 104 mit einer Bezugsziffer versehen sind.

Schaltungstechnisch kann die Sache so ausgestaltet sein, dass der Positionssensor 98 für das Element 94 detektiert, der Positionssensor 100 für das Element 88, der Positionssensor 102 für das Element 96 und der Positionssensor 104 für das Element 90.

Werden Endoskopteil 72 und Lichtzuführteil 74 in den in den Figuren 3 und 4 dargestellten Positionen miteinander gekuppelt, so kommt der Querschnittsbereich des Lichtleiters 78 auf insgesamt vier lichtabstrahlenden Elementen zum Liegen, nämlich auf den benachbarten lichtabstrahlenden Elementen 88 und 90 des inneren Beleuchtungsrings und den lichtabstrahlenden Elementen 94 und 96 des äußeren Beleuchtungsringes (siehe gestrichelter Kreis in Figur 3).

Diese Position kann, wie zuvor beschrieben, über den Positionsgeber 82 detektiert werden, so dass in diesem Fall dann die vier lichtabstrahlenden Elemente 88, 90, 94 und 96 aktiviert werden.

Wird das Endoskopteil 72 beispielsweise im Uhrzeigersinn etwas verdreht, tritt dann eine Situation ein, in der der Querschnitt des Lichtleiters 78 nur noch auf den lichtabstrahlenden Elementen 90 des inneren und 96 des äußeren Ringes zum Liegen kommt.

Auch dies ist wieder über die Positionssensoren erfassbar, so dass dann nur noch diese beiden lichtabstrahlenden Elemente 90 und 96 aktiviert werden.

Dies zeigt nicht nur wieder die besonders ökonomische Beleuchtungslichtführung und Aktivierung, sondern eröffnet auch weitere Möglichkeiten. So ist es bei einer solchen Ausgestaltung mit mehreren Ringen möglich, mit mehreren lichtabstrahlenden Elementen unterschiedlicher Farbe zu arbeiten. Dies kann beispielsweise bei einer photodynamischen Diagnose sinnvoll sein, wenn beispielsweise einmal nur mit weißem Licht gearbeitet werden soll, um den Untersuchungsbereich allgemein zu inspizieren, für eine photodynamische Diagnose dann aber beispielsweise nur ein Rot- oder Blaulicht eingestrahlt werden soll, um ein speziell präpariertes Gewebe, meist ein Tumorgewebe, zu identifizieren.

Dies ist nun einfach dadurch zu realisieren, dass die lichtabstrahlenden Elemente beispielsweise LEDs oder auch OLEDs unterschiedlicher Farbabstrahlung sind.

Befindet sich beispielsweise der Lichtleiter 78 in der zuvor beschriebenen etwas verdrehten Position, in der er nur die beiden lichtabstrahlenden Elemente 96 und 90 belegt, könnte man eines der lichtabstrahlenden Elemente nur mit Weißlicht betreiben, das andere aber mit einem farbigen Licht, so dass in einer bestimmten Stellung wechselweise mit unterschiedlichem Licht gearbeitet werden kann. Es sind dann entsprechende Schaltungen oder Tastaturen vorgesehen, um dies zu bewerkstelligen bzw. zu steuern.

Es ist denkbar, dass in das Lichtzuführteil 74 von Figur 3 auch das Endoskopteil 12 von Figur 2 eingeschoben werden kann. Je nach Lage der Lichtleiter 50 und 52 des Endoskopteiles 12 kommen diese beispielsweise ausschließlich mit dem Bereich von lichterzeugenden Elementen des ersten inneren Ringes 86 oder des zweiten äußeren Ringes 92 in Berührung. Man kann die Position der Lichtleiter 50 und 52 auch so wählen, dass einer immer nur mit den Lichtleitern des äußeren Ringes, der andere nur mit den Lichtleitern des inneren Ringes in Ausrichtung steht. Man kann so schalten, dass der eine Lichtleiter 50 beispielsweise mit Weißlicht betreibbar ist, der zweite Lichtleiter 52 mit einem für eine photodynamische Diagnose geeigneten Beleuchtungslicht.

Dies zeigt erneut die hohe Flexibilität des Videoendoskopkuppelsystems.

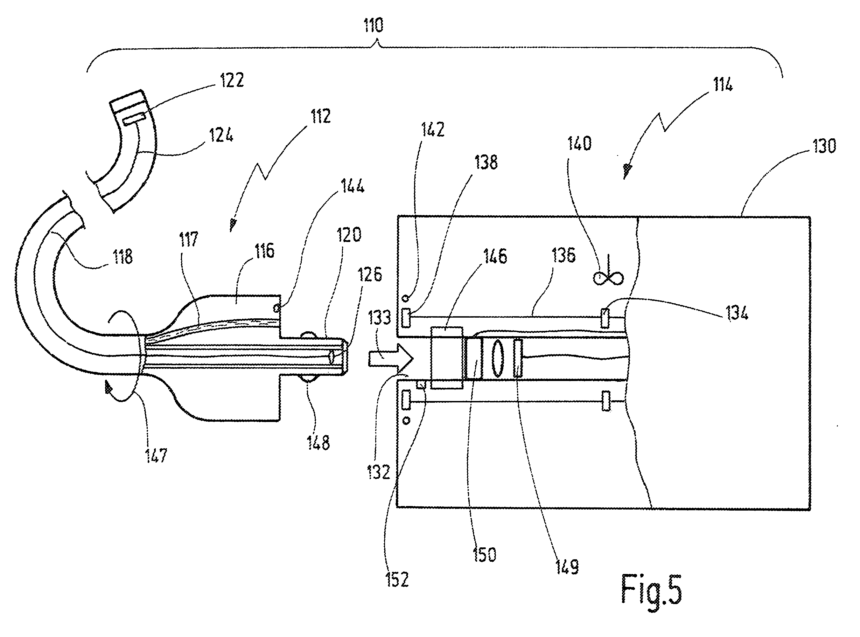

In Figur 5 ist ein drittes Ausführungsbeispiel eines Videoendoskopes dargestellt, das in seiner Gesamtheit mit der Bezugsziffer 110 bezeichnet ist.

Das Videoendoskop 110 weist ein Endoskopteil 112 und ein Lichtzuführteil 114 auf.

Das Endoskopteil 112 ist als flexibles Endoskop ausgeführt, und weist einen Kopf 116 auf, der in einen flexiblen Schaft 118 übergeht. Vom Kopf 116 steht dann wieder ein Zapfen 120 vor, der die zuvor beschriebene Aufgabe bzw. Funktion hat. Auch hier ist ein flexibler Lichtleiter 117 bis zur Kupplungsfläche am Kopf 116 geführt.

In dem dargestellten Ausführungsbeispiel ist das Endoskopteil 112 als Videoendoskop ausgebildet. D.h., im Bereich seines distalen Endes ist ein elektrischer Bildwandler 122 angeordnet, der das einfallende optische Signal in ein elektrisches Signal umwandelt. Dieses elektrische Signal wird über eine Leitung 124 bis zu einem Anschluss 126 im Zapfen 120 geführt.

Das Lichtzuführteil 114 ist als ein Standgerät 130 ausgestaltet.

Auch hier ist eine Aushöhlung 132 vorhanden, in die, wie zuvor beschrieben, der Zapfen 120 des Endoskopteils 112 eingeschoben werden kann, wie das durch einen Pfeil 133 angedeutet ist. In dem Standgerät 130 ist ein jeweiliges lichtabstrahlendes Element 138 so aufgebaut, dass es mit einer weiter im Inneren des Gerätes 130 liegenden Halbleiterlichtquelle 134 über einen Lichtleiter 136 verbunden ist. Dies eröffnet die Möglichkeit, insbesondere bei zahlreichen und lichtintensiv abstrahlenden Halbleiterlichtquellen 134 diese über eine Kühlung 140 zu kühlen.

Dem lichtabstrahlenden Element 138 ist wieder ein Positionssensor 142 zugeordnet, der mit dem entsprechenden Positionsgeber 144 am Endoskopteil 112 zusammenwirkt, wie das zuvor beschrieben ist.

Im Bereich der Aushöhlung 132 ist ein Antrieb 146 angeordnet, über den ein in das Standgerät 130 eingestecktes Endoskopteil 112 gedreht werden kann, wie das durch einen Pfeil 147 dargestellt ist.

Damit in das Lichtzuführteil 114 ein Endoskopteil eingeschoben werden kann, das das Bild optisch leitet, ist ein elektrischer Bildwandler 149 vorgesehen.

Damit das Lichtzuführteil 114 auch mit solchen Endoskopen gekoppelt werden kann, die schon als Videoendoskop ausgebildet sind, ist im Bereich der Aushöhlung 132 ein Stecker 150 vorgesehen, um das elektrische Signal vom Anschluss 126 des Endoskopteiles 112 entsprechend weiterzuleiten.

Auch das zeigt die hohe Flexibilität der Konstruktion, d.h. das Lichtzuführteil 114 kann mit unterschiedlichen Endoskoptypen gekoppelt werden.

In der Aushöhlung 132 ist ein Leseelement 152 angeordnet, das entsprechende Informationen, beispielsweise aus einem Identifikationselement am Zapfen 120, ablesen kann.

An der Außenseite des Zapfens 120 sind hier federelastische Rasten 148 vorgesehen, die ein Verrasten beispielsweise mit dem Antrieb 146 erlauben.

Dadurch ist nicht nur eine Abzugsicherung gewährleistet, sondern auch gleich eine mechanisch drehfeste Verbindung zwischen dem Zapfen 120 und dem Antrieb 146 geschaffen.

Das Schließen und Lösen der Kopplung zwischen Endoskopteil 112 und Lichtzuführteil 114 funktioniert wie zuvor beschrieben, also Einstecken bzw. Abziehen des Zapfens 120 in oder vom Standgerät 130.

## Patentansprüche

1. Videoendoskop, mit einem Endoskopteil (12, 72, 112), das proximalseitig einen mittigen Bildsignalleiter (48) und zumindest einen Lichtleiter (50, 52; 78; 117) aufweist, und mit einem an das Endoskopteil (12, 72, 112) koppelbaren Lichtzuführteil (14, 74, 114), das distalseitig einen mittigen Bildsignalleiteranschluss (22) sowie eine Lichtzuführung aufweist, die im Koppelbereich koaxial um den Bildsignalleiteranschluss (22) angeordnet ist, wobei Endoskopteil (12, 72, 112) und Lichtzuführteil (14, 74, 114) drehbar zueinander sind, **dadurch gekennzeichnet, dass** das Lichtzuführteil (14, 74, 114) mehrere, um den Bildsignalleiteranschluss (22) herum angeordnete lichtabstrahlende Elemente (26, 28; 88, 90, 94, 96; 138) aufweist, die jeweils über schaltbare Halbleiterlichtquellen (134) versorgbar sind, wobei im Bereich der Elemente (26, 28; 88, 90, 94, 96; 138) Positionssensoren (34, 36; 98, 100, 102) angeordnet sind, und dass am Endoskopteil (12, 72, 112) im Bereich eines Lichtleiters (50, 52; 78; 117) ein Positionsgeber (58, 60; 82; 144) angeordnet ist, dessen Position in gekoppeltem Zustand durch die Positionssensoren (34, 36; 38, 100, 102) erfassbar ist und dass die lichtabstrahlenden Elemente (26,28;88,90,94,96;138) ausgebildet sind **dadurch** zumindest dem Lichtleiter (5052;78;117) gegenüberliegend aktiviert zu werden.

2. Videoendoskop nach Anspruch 1, **dadurch gekennzeichnet, dass** die lichtabstrahlenden Elemente (26, 28; 88, 90, 94, 96; 138) ausgebildet sind nur im Querschnittsbereich eines Lichtleiters (50, 52; 78; 117) des Endoskopteiles (12, 72, 112) liegend aktiviert zu werden.

3. Videoendoskop nach Anspruch 1 oder 2, **dadurch gekennzeichnet, dass** die lichtabstrahlenden Elemente (26, 28; 88, 90, 94, 96; 138) segmentartig ausgebildet sind.

4. Videoendoskop nach einem der Ansprüche 1 bis 3, **dadurch gekennzeichnet, dass** die lichtabstrahlenden Elemente (26, 28; 88, 90, 94, 96; 138) zu zumindest einem Ring (30; 86; 92) zusammengesetzt sind.

5. Videoendoskop nach einem der Ansprüche 1 bis 4, **dadurch gekennzeichnet, dass** die schaltbaren Halbleiterlichtquellen (134) ausgewählt sind aus der Gruppe bestehend aus LEDs, OLEDs, Diodenlaser oder Kombinationen davon.

6. Videoendoskop nach einem der Ansprüche 1 bis 5, **dadurch gekennzeichnet, dass** die schaltbare Halbleiterlichtquelle (134) unmittelbar das lichtabstrahlende Element (26, 28; 88, 90, 94, 96; 138) bildet.

7. Videoendoskop nach einem der Ansprüche 1 bis 5, **dadurch gekennzeichnet, dass** die schaltbaren Halbleiterlichtquellen (134) im Abstand zu den endseitigen lichtabstrahlenden Elementen (138) angeordnet sind und mit diesen über Lichtleiter (136) verbunden sind.

8. Videoendoskop nach einem der Ansprüche 1 bis 7, **dadurch gekennzeichnet, dass** die lichtabstrahlenden Elemente (26, 28; 88, 90, 94, 96; 138) Licht unterschiedlicher Farbe abstrahlen.

9. Videoendoskop nach einem der Ansprüche 1 bis 8, **dadurch gekennzeichnet, dass** die Halbleiterlichtquellen (134) in einer separaten Aparatur aufgenommen sind.

10. Videoendoskop nach einem der Ansprüche 1 bis 9, **dadurch gekennzeichnet, dass** eine Kühlung (140) zum Kühlen der Halbleiterlichtquellen (134) vorhanden ist.

11. Videoendoskop nach einem der Ansprüche 1 bis 10, **dadurch gekennzeichnet, dass** die Positionssensoren (34, 36; 38, 100, 102) und die Positionsgeber (58, 60; 82; 144) als Hall-Sensoren ausgebildet sind.

12. Videoendoskop nach einem der Ansprüche 1 bis 11, **dadurch gekennzeichnet, dass** das Endoskopteil (12, 72, 112) ein Identifikationselement (49) aufweist, welches über ein im Lichtzuführteil (14, 74, 114) angeordnetes Leseelement (152) auslesbar ist.

13. Videoendoskop nach einem der Ansprüche 1 bis 12, **dadurch gekennzeichnet, dass** ein Rastmechanismus vorgesehen ist, der ein Verrasten von Endoskopteil (12) und Lichtzuführteil (14) in gekoppeltem Zustand bewirkt.

14. Videoendoskop nach Anspruch 13, **dadurch gekennzeichnet, dass** der Rastmechanismus derart ausgebildet ist, dass ein Drehen des Endoskopteiles (12) um das Lichtzuführteil (14) in Positionen mit äquidistanten Winkelstellungen möglich ist.

15. Videoendoskop nach einem der Ansprüche 1 bis 14, **dadurch gekennzeichnet, dass** das Endoskopteil (12) ein Steuerelement (66) aufweist, über das es bei erfasstem Lichtzuführteil (14) um dieses drehbar ist.

16. Videoendoskop nach einem der Ansprüche 1 bis 15, **dadurch gekennzeichnet, dass** das Lichtzuführteil (14) als Kamerateil mit einem elektrischen Bildwandler ausgebildet ist.

17. Videoendoskop nach einem der Ansprüche 1 bis 16, **dadurch gekennzeichnet, dass** im Endoskopteil (112) ein elektrischer Bildwandler (122) integriert ist, und dass im Lichtzuführteil (114) ein Steckerteil (150) vorgesehen ist, durch das das elektrische Bildsignal weiterleitbar ist.

18. Videoendoskop nach einem der Ansprüche 1 bis 17, **dadurch gekennzeichnet, dass** das Lichtzuführteil (114) als Standgerät (130) ausgebildet ist, in das das Endoskopteil (112) einsteckbar ist.

19. Videoendoskop nach einem der Ansprüche 1 bis 18, **dadurch gekennzeichnet, dass** im Lichtzuführteil (114) ein Antrieb (146) vorhanden ist, durch den ein angekoppeltes Endoskopteil (112) drehbar ist.

20. Videoendoskop nach einem der Ansprüche 1 bis 19, **dadurch gekennzeichnet, dass** das Endoskopteil (12, 22, 112) einen mittigen, nach proximal vorstehenden Zapfen (46, 76, 120) aufweist, in dem der Bildsignalleiter aufgenommen ist.

21. Videoendoskop nach Anspruch 20, **dadurch gekennzeichnet, dass** der Bildsignalleiteranschluss (22) des Lichtzuführteiles (14, 74, 114) eine Aushöhlung (24, 84, 132) aufweist, in die der vorstehende Zapfen (46, 71, 120) einsteckbar ist.

## Claims

1. Video endoscope, comprising an endoscope part (12, 72, 112), which has proximally a central image signal conductor (48) and at least one optical waveguide (50, 52; 78; 117), and comprising a light feeding part (14, 74, 114), which can be coupled to the endoscope part (12, 72, 112) and which has distally a central image signal conductor connection (22) and a light feed arranged coaxially around the image signal conductor connection (22) in the coupling region, wherein endoscope part (12, 72, 112) and light feeding part (14, 74, 114) are rotatable with respect to one another, **characterized in that** the light feeding part (14, 74, 114) has a plurality of light-emitting elements (26, 28; 88, 90, 94, 96; 138) which are arranged around the image signal conductor connection (22) and which can be supplied in each case by means of switchable semiconductor light sources (134), wherein position sensors (34, 36; 98, 100, 102) are arranged in the region of the elements (26, 28; 88, 90, 94, 96; 138), and **in that** a position transmitter (58, 60; 82; 144) is arranged on the endoscope part (12, 72, 112) in the region of an optical waveguide (50, 52; 78; 117), the position of which position transmitter in the coupled state can be detected by position sensors (34, 36; 38, 100, 102), and **in that** at least light-emitting elements (26, 28; 88, 90, 94, 96; 138) lying opposite the optical waveguide (50, 52; 78; 117) are thereby activated.

2. Video endoscope of Claim 1, **characterized in that** only those light-emitting elements (26, 28; 88, 90, 94, 96; 138) which lie in the cross-sectional region of an optical waveguide (50, 52; 78; 117) of the endoscope part (12, 72, 112) are activated.

3. Video endoscope of Claims 1 or 2, **characterized in that** the light-emitting elements (26, 28; 88, 90, 94, 96; 138) are embodied in segment-like fashion.

4. Video endoscope of anyone of Claims 1 to 3, **characterized in that** the light-emitting elements (26, 28; 88, 90, 94, 96; 138) are combined to form at least one ring (30; 86; 92).

5. Video endoscope of anyone of Claims 1 to 4, **characterized in that** the switchable semiconductor light sources (134) are selected from the group consisting of LEDs, OLEDs, diode lasers or combinations thereof.

6. Video endoscope of anyone of Claims 1 to 5, **characterized in that** the switchable semiconductor light source (134) directly forms the light-emitting element (26, 28; 88, 90, 94, 96; 138).

7. Video endoscope of anyone of Claims 1 to 5, **characterized in that** the switchable semiconductor light sources (134) are arranged at a distance from the light-emitting elements (138) situated on the end side and are connected to said elements via optical waveguides (136).

8. Video endoscope of anyone of Claims 1 to 7, **characterized in that** the light-emitting elements (26, 28; 88, 90, 94, 96; 138) emit light of different colours.

9. Video endoscope of anyone of Claims 1 to 8, **characterized in that** the semiconductor light sources (134) are accommodated in a separate apparatus.

10. Video endoscope of anyone of Claims 1 to 9, **characterized in that** a cooling system (140) for cooling the semiconductor light sources (134) is present.

11. Video endoscope of anyone of Claims 1 to 10, **characterized in that** the position sensors (34, 36; 38, 100, 102) and the position transmitters (58, 60; 82; 144) are embodied as Hall sensors.

12. Video endoscope of anyone of Claims 1 to 11, **characterized in that** the endoscope part (12, 72, 112) has an identification element (49), which can be read by means of a reading element (152) arranged in the light feeding part (14, 74, 114).

13. Video endoscope of anyone of Claims 1 to 12, **characterized in that** a latching mechanism is provided, which brings about a latching of endoscope part (12) and light feeding part (14) in the coupled state.

14. Video endoscope of Claim 13, **characterized in that** the latching mechanism is embodied in such a way that it is possible for the endoscope part (12) to rotate about the light feeding part (14) in positions with equidistant angular locations.

15. Video endoscope of anyone of Claims 1 to 14, **characterized in that** the endoscope part (12) has a control element (66), by means of which, when the light feeding part (14) is detected, said endoscope part can be rotated about said light feeding part.

16. Video endoscope of anyone of Claims 1 to 15, **characterized in that** the light feeding part (14) is embodied as a camera part with an electrical image converter.

17. Video endoscope of anyone of Claims 1 to 16, **characterized in that** an electrical image converter (122) is integrated in the endoscope part (112) and **in that** a connector part (150) is provided in the light feeding part (114), by means of which connector part the electrical image signal can be forwarded.

18. Video endoscope of anyone of Claims 1 to 17, **characterized in that** the light feeding part (114) is embodied as a standing unit (130), into which the endoscope part (112) can be inserted.

19. Video endoscope of anyone of Claims 1 to 18, **characterized in that** a drive (146) is present in the light feeding part (114), by means of which drive a coupled endoscope part (112) can be rotated.

20. Video endoscope of anyone of Claims 1 to 19, **characterized in that** the endoscope part (12, 22, 112) has a central, proximally projecting pin (46, 76, 120), which accommodates the image signal conductor.

21. Video endoscope of Claim 20, **characterized in that** the image signal conductor connection (22) of the light feeding part (14, 74, 114) has a cavity (24, 84, 132), into which the projecting pin (46, 71, 120) can be inserted.

## Revendications

1. Vidéo-endoscope doté d'une partie endoscope (12, 72, 112) qui présente côté proximal un conducteur de signal d'image (48) central et au moins un conducteur de lumière (50, 52 ; 78 ; 117), et d'une partie amenée de lumière (14, 74, 114) pouvant être couplée à la partie endoscope (12, 72, 112), qui présente côté distal un raccord de conducteur de signal d'image (22) central ainsi qu'une amenée de lumière qui est disposée coaxialement autour du raccord de conducteur de signal d'image (22) dans la zone de couplage, partie endoscope (12, 72, 112) et partie amenée de lumière (14, 74, 114) pouvant tourner l'une par rapport à l'autre, **caractérisé en ce que** la partie amenée de lumière (14, 74, 114) présente plusieurs éléments émetteurs de lumière (26, 28 ; 88, 90, 94, 96 ; 138) disposés autour du raccord de conducteur de signal d'image (22), qui peuvent être alimentés chacun par des sources de lumière à semi-conducteurs commutables (134), des capteurs de position (34, 36 ; 98, 100, 102) étant disposés dans la région des éléments (26, 28 ; 88, 90, 94, 96 ; 138), et qu'un transmetteur de position (58, 60 ; 82 ; 144) est disposé sur la partie endoscope (12, 72, 112) dans la région d'un conducteur de lumière (50, 52 ; 78 ; 117), dont la position dans l'état couplé peut être détectée par les capteurs de position (34, 36 ; 38, 100, 102), et que les éléments émetteurs de lumière (26, 28 ; 88, 90, 94, 96 ; 138) sont conçus pour être activés de ce fait au moins quand ils sont situés en face du conducteur de lumière (50, 52 ; 78 ; 117).

2. Vidéo-endoscope selon la revendication 1, **caractérisé en ce que** les éléments émetteurs de lumière (26, 28 ; 88, 90, 94, 96 ; 138) sont conçus pour être activés seulement quand ils sont situés dans la section transversale d'un conducteur de lumière (50, 52 ; 78 ; 117) de la partie endoscope (12, 72, 112).

3. Vidéo-endoscope selon la revendication 1 ou 2, **caractérisé en ce que** les éléments émetteurs de lumière (26, 28 ; 88, 90, 94, 96 ; 138) sont conçus à la manière de segments.

4. Vidéo-endoscope selon une des revendications 1 à 3, **caractérisé en ce que** les éléments émetteurs de lumière (26, 28 ; 88, 90, 94, 96 ; 138) sont assemblés de manière à former au moins un anneau (30 ; 86 ; 92).

5. Vidéo-endoscope selon une des revendications 1 à 4, **caractérisé en ce que** les sources de lumière à semi-conducteurs commutables (134) sont sélectionnées dans le groupe composé des LED, OLED, diodes laser ou de combinaisons de celles-ci.

6. Vidéo-endoscope selon une des revendications 1 à 5, **caractérisé en ce que** la source de lumière à semi-conducteurs commutable (134) forme directement l'élément émetteur de lumière (26, 28 ; 88, 90, 94, 96 ; 138).

7. Vidéo-endoscope selon une des revendications 1 à 5, **caractérisé en ce que** les sources de lumière à semi-conducteurs commutables (134) sont disposées à distance des éléments émetteurs de lumière (138) situés à l'extrémité et reliées à ceux-ci par des conducteurs de lumière (136).

8. Vidéo-endoscope selon une des revendications 1 à 7, **caractérisé en ce que** les éléments émetteurs de lumière (26, 28 ; 88, 90, 94, 96 ; 138) émettent une lumière de couleur différente.

9. Vidéo-endoscope selon une des revendications 1 à 8, **caractérisé en ce que** les sources de lumière à semi-conducteurs (134) sont montées dans un appareillage séparé.

10. Vidéo-endoscope selon une des revendications 1 à 9, **caractérisé en ce qu'**un refroidissement (140) pour refroidir les sources de lumière à semi-conducteurs (134) est prévu.

11. Vidéo-endoscope selon une des revendications 1 à 10, **caractérisé en ce que** les capteurs de position (34, 36 ; 38, 100, 102) et les transmetteurs de position (58, 60 ; 82 ; 144) sont réalisés sous la forme de capteurs à effet Hall.

12. Vidéo-endoscope selon une des revendications 1 à 11, **caractérisé en ce que** la partie endoscope (12, 72, 112) présente un élément d'identification (49), lequel peut être lu par un élément de lecture (152) disposé dans la partie amenée de lumière (14, 74, 114).

13. Vidéo-endoscope selon une des revendications 1 à 12, **caractérisé en ce qu'**il est prévu un mécanisme d'encliquetage qui entraîne un encliquetage de la partie endoscope (12) et de la partie amenée de lumière (14) à l'état couplé.

14. Vidéo-endoscope selon la revendication 13, **caractérisé en ce que** le mécanisme d'encliquetage est conçu de façon qu'une rotation de la partie endoscope (12) autour de la partie amenée de lumière (14) dans des positions angulaires équidistantes soit possible.

15. Vidéo-endoscope selon une des revendications 1 à 14, **caractérisé en ce que** la partie endoscope (12) présente un élément de commande (66) au moyen duquel on peut la faire tourner autour de la partie amenée de lumière (14) lorsque celle-ci est saisie.

16. Vidéo-endoscope selon une des revendications 1 à 15, **caractérisé en ce que** la partie amenée de lumière (14) est réalisée sous la forme d'une partie caméra avec un convertisseur d'image électrique.

17. Vidéo-endoscope selon une des revendications 1 à 16, **caractérisé en ce qu'**un convertisseur d'image électrique (122) est intégré dans la partie endoscope (112), et qu'il est prévu dans la partie amenée de lumière (114) une partie connecteur (150) à travers laquelle le signal d'image électrique peut être transmis.

18. Vidéo-endoscope selon une des revendications 1 à 17, **caractérisé en ce que** la partie amenée de lumière (114) est réalisée sous la forme d'un appareil à poser (130) dans lequel la partie endoscope (112) est enfichable.

19. Vidéo-endoscope selon une des revendications 1 à 18, **caractérisé en ce qu'**il est prévu dans la partie amenée de lumière (114) un entraînement (146) au moyen duquel on peut faire tourner une partie endoscope (112) accouplée.

20. Vidéo-endoscope selon une des revendications 1 à 19, **caractérisé en ce que** la partie endoscope (12, 22, 112) présente un tenon (46, 76, 120) central, en saillie en direction proximale, dans lequel le conducteur de signal d'image est reçu.

21. Vidéo-endoscope selon la revendication 20, **caractérisé en ce que** le raccord de conducteur de signal d'image (22) de la partie amenée de lumière (14, 74, 114) présente une cavité (24, 84, 132) dans laquelle le tenon (46, 71, 120) en saillie est enfichable.
